# EUROPEAN PATENT SPECIFICATION

(11) **EP 0 998 936 B1**
(45) Date of publication and mention of the grant of the patent: **15.03.2006**
(21) Application number: 98118558.0
(22) Date of filing: 01.10.1998
(51) Int. Cl.: A61K 31/715, C12Q 1/68, A61P 43/00

(54) **Substances for the prevention of pathological overexpression of TGF-Beta and methods for evaluating same**
Substanzen zur Vorbeugung von krankhafter TGF-beta Überexpression und Methoden derer Ermittlung
Composés pour prévenir la surexpression du facteur de croissance transformant beta et méthodes pour les déterminer

(43) Date of publication of application: 10.05.2000
(73) Proprietor: Gambaro, Giovanni, 30037 Scorzè (Venezia) (IT); Schleicher, Erwin, 80995 München (DE)
(72) Inventor: Gambaro, Giovanni, 30037 Scorzè (Venezia) (IT); Schleicher, Erwin, 80995 München (DE)
(74) Representative: Olgemöller, Luitgard Maria

(56) References cited:
- EP-A- 0 629 697
- WO-A-90/06755
- WO-A-95/19987
- CEOL, M. (1) ET AL: "Effect of heparin treatment on glomerular collagen IV alpha-1 and TGF - beta -1 expression and deposition in diabetic rats." DIABETOLOGIA, (1995) VOL. 38, NO. SUPPL. 1, PP. A62. MEETING INFO.: 31ST ANNUAL MEETING OF THE EUROPEAN ASSOCIATION FOR THE STUDY OF DIABETES STOCKHOLM, SWEDEN SEPTEMBER 12-16, 1995 ISSN: 0012-186X., XP002093994
- HAGEGE, JACQUELINE ET AL: "Heparin selectively inhibits synthesis of tissue type plasminogen activator and matrix deposition of plasminogen activator inhibitor 1 by human mesangial cells" LAB. INVEST. (1994), 71(6), 828-37 CODEN: LAINAW;ISSN: 0023-6837, XP002093995
- YOUNG B.A. ET AL: "Cellular events in the evolution of experimental diabetic nephropathy." ISSN: 0085-2538 CODEN: KDYIA5, XP002093996 United States
- YOKOYAMA H. ET AL: "Central role of TGF -. beta. in the pathogenesis of diabetic nephropathy and macrovascular complications: A hypothesis." DIABETIC MEDICINE, (1996) 13/4 (313-320). ISSN: 0742-3071 CODEN: DIMEEV, XP002093997 United Kingdom
- TAMEHIRO, KAZUHITO: "The effect of low molecular weight heparin on implanted cardiac allograft vascular disease. The study with RT-PCR method" KURUME IGAKKAI ZASSHI (1994), 57(12), 1284-93 CODEN: KIZAAL;ISSN: 0368-5810, XP002093998
- IOCONO, JOSEPH A. ET AL: "Inhibiting the differentiation of myocardiocytes by hyaluronic acid" J. SURG. RES. (1998), 76(2), 111-116 CODEN: JSGRA2;ISSN: 0022-4804, XP002093999
- SNOW, ALAN D. ET AL: "Heparin modulates the composition of the extracellular matrix domain surrounding arterial smooth muscle cells" AM. J. PATHOL. (1990), 137(2), 313-30 CODEN: AJPAA4;ISSN: 0002-9440, XP002094000
- HOFFMAN, RICHARD ET AL: "Selective inhibition of cell proliferation and DNA synthesis by the polysulfated carbohydrate.iota.-carrageenan" CANCER CHEMOTHER. PHARMACOL. (1995), 36(4), 325-34 CODEN: CCPHDZ;ISSN: 0344-5704, XP002094001
- KARDAMI, ELISSAVET ET AL: "Heparin inhibits skeletal muscle growth in vitro" DEV. BIOL. (1988), 126(1), 19-28 CODEN: DEBIAO;ISSN: 0012-1606, XP002094002
- FUKUDA K. ET AL: "Hyaluronic acid increases proteoglycan synthesis in bovine articular cartilage in the presence of interleukin-1." JOURNAL OF PHARMACOLOGY AND EXPERIMENTAL THERAPEUTICS, (1996) 277/3 (1672-1675). REFS: 29 ISSN: 0022-3565 CODEN: JPETAB, XP002094003 United States
- LIDER O ET AL: "Inhibition of T lymphocyte heparanase by heparin prevents T cell migration and T cell-mediated immunity." EUROPEAN JOURNAL OF IMMUNOLOGY, (1990 MAR) 20 (3) 493-9. JOURNAL CODE: EN5. ISSN: 0014-2980., XP002094004 GERMANY, WEST: Germany, Federal Republic of
- TASAKI, HIROMI ET AL: "Promotion of aortic smooth muscle cell proliferation by hypercholesterolemic LDL and its suppression by heparin or sulfated glycosaminoglycans" CELL. PHYSIOL. BIOCHEM. (1994), 4(1-2), 57-71 CODEN: CEPBEW;ISSN: 1015-8987, XP002094005
- LANZILLO J ET AL: "A competitive quantitative polymerase chain reaction assay for bovine transforming factor B1 mRNA" LIFE SCIENCES, vol. 59, no. 25/26, 15 November 1996 (1996-11-15), pages 2157-65, XP002114734
- ROBERTS A ET AL: "Multiple forms of TGF-beta: distinct promoters and differential expression" CIBA FOUNDATION SYMPOSIUM 57, vol. 157, 1991, page 7-15 XP002114735
- KANAME S ET AL: "Autocrine secretion of transforming growth factor-beta in cultured rat mesangial cells" KIDNEY INTERNATIONAL, vol. 42, December 1992 (1992-12), pages 1319-27, XP002114736
- KIGUCHI K ET AL: "Elevation of transforming growth factor-alpha mRNA and protein expression by diverse tumor promoters in SENCAR mouse epidermis" MOLECULAR CARCINOGENESIS, vol. 12, April 1995 (1995-04), pages 225-35, XP002114737
- YAO J ET AL: "Role of transforming growth factor-beta in the growth inhibition of cultured rat mesangial cells in high glucose media" CHINESE MEDICAL JOURNAL, vol. 107, no. 6, June 1996 (1996-06), pages 455-9, XP002114738

## Description

This invention relates to the inhibition of disease- or repair-induced overexpression of growth factors, particularly transforming growth factor β (TGF-β).

A variety of cytokines and growth factors have been suggested to play a role in the development of organ fibrosis however, the multifunctional cytokine TGF-β appears to be a key prosclerotic factor. Three TGF-β isoforms, termed TGF-β1, TGF-β2 and TGF-β3, have been identified in mammals. These isoforms exhibit both overlapping and distinct activities, but TGF-β1 seems to play the main role, due to both its activity and its presence. The TGF-β family is a multifuncional cytokine family since it is involved e.g. in the differentiation in embryonic development, in the regulation of cell proliferation and it controls the production of extracellular matrix proteins by stimulating the de novo synthesis of the matrix components (collagen and proteoglycans) and by inhibiting matrix degradation. Furthermore, TGF-β exerts immunosupressive and chemotactic activities and, by auto-/paracrine action, it may induce other cells to synthesize and secrete cytokines and growth factors including TGF-β. Studies using i) proteins that bind TGF-β and neutralize its activity like TGF-β antibodies or decorin (which specifically binds TGF-β) or ii) isoform specific TGF-β antisense oligonucleotides which inhibit the synthesis of the respective TGF-β isoform or iii) target-directed overexpression of TGF-β1 leading to excessive fibrosis/sclerosis of the organ indicate the importance specifically of TGF-β1 in the fibrotic/sclerotic processes. In addition, in most human diseases associated with fibrosis/sclerosis, TGF-β1 overexpression has been found. Overexpression of TGF-β in early stages of Duchenne muscular dystrophy has also been found to be critical for the development of muscle fibrosis in these patients. These data indicate the causal involvement of TGF-β1 in the initiation and/or progression of fibrotic processes.

TGF-β1 is synthesized as a TGF-β1 precursor protein consisting of 391 amino acids. After proteolytic cleavage of the C-terminus of the TGF-β1 (112 amino acids) the 25-kDa homodimer forms the bioactive protein. However, if the N-terminal remnant of the precursor, denoted as the TGF-β1 latency associated peptide (LAP), is associated to mature TGF-β1, it blocks bioactivity. In most organs the latent form of TGF-β1 is composed of three distinct components; mature TGF-β1, LAP and latent TGF-β1 binding protein (LTBP). Although several mechanisms for the in vivo activation of TGF-β have been proposed this issue remains to be clarified.

TGF-β1 protein can be found in normal tissues by immunohistochemical studies in variable amounts mostly in low levels in parenchymal cells. Very high levels of TGF-β1 protein are particularly found in platelets (needed for acute wound repair) and in bones. While TGF-β1 synthesis is low in most normal parenchymal cells it is mostly increased after pathological condition/situation occurs. Therefore, in addition to the TGF-β1 protein e.g. from platelets eventually needed for acute repair processes, TGF-β expression is also induced in other pathological processes and for unknown reasons not terminated which lead to chronic TGF-β production/action with its potentially undesired side action like organ fibrosis.

Studies on the regulation of the TGF-β1 gene expression showed that two distinct regions of the TGF-β1 promotor are involved in expression. These regions bind two different transcription factors e.g. stimulatory protein 1 (Sp1) and activator protein 1 (AP1). While Sp1 sites control basal TGF-β1 transcription activation, activation of AP-1 induces TGF-β, particularly TGF-β1, gene overexpression (Kim et al. *J. Biol. Chem*. 264: 19373-19378, 1989). The AP-1 mediated TGF-β1 expression may be stimulated by phorbolester myristate acetate (PMA).

Pathological overexpression of TGF-β causes
1. excessive accumulation of extracellular matrix (ECM) eventually leading to fibrosis and subsequent organ failure
2. inhibition of cell proliferation thus possibly interfering with physiological repair mechanism
3. immunomodulation and immunosuppression

ad 1) A survey by the Department of Health and Human Services revealed that approximately 45% of all deaths in the US during 1984-1989 were due to fibrotic diseases. Although the largest contributor to this category was atherosclerosis, fibrotic diseases of the lung, liver and kidney account for the majority of the rest of the cases. Fibrosis (sclerosis is synonymously used) of an organ is caused by progressive deposition of ECM initiated by an acute or chronic stimulus. For unknown reasons or because the pathological stimulus is still active (e.g. chronic infection, persistently increased glucose, alcohol or cholesterol levels) the initially reparative process is not down-regulated and the chronic matrix production and deposition continues. Chronic upregulation of the prosclerotic transforming growth factor β (TGF β) has been shown to be a key mediator in the development of organ fibrosis. The involvement of TGF-β has been particularly demonstrated for the development of diabetic and other chronic nephropathies, fibrosis of the liver, lung and other organs. Furthermore, in contrast to embryonal wound healing, at the adult wound site TGF-β is present at high levels for the duration of healing and beyond indicating overexpression of TGF-β (Martin, *Science,* 276: 75-81, 1997).
ad 2) Besides the promotion of fibrosis TGF-β exhibits strong antiproliferative activity in most untransformed cells e.g. epithelial, endothelial and mesenchymal cells. Therefore, in repair processes an unwanted inhibition of parenchymal cell proliferation occurs if local TGF-β overexpression is induced. Thus the regeneration of the affected organ is limited by the antiproliferative activity of TGF-β (Böttinger et al. *Proc. Natl. Acad. Sci. USA*. 93:5877-5882, 1996).
ad 3) TGF-β exerts immunosuppressive and immunomodulatory activities. In part the immunosuppressive effects may derive from their antiproliferative properties (e.g. inhibition of proliferation of T- and B-lymphocytes). Furthermore, TGF-β inhibits mitogen-stimulated production of IL-2,-6,-10 in peripheral human T-lymphocytes (Reinhold et al. *J. Immunol. Methods* 209: 203-206, 1997). Therefore, e.g. tumor-induced TGF-β overexpression may protect the tumor cells from attack of the immune system. This has been particularly shown for glioblastoma cells. On the other hand TGF-β is a potent chemoattractant for monocytes/macrophages which are involved in the local inflammatory and immunological reaction, and, particularly in the prostatic cancer, might increase the aggressivity of this tumor because of the secretion of angiogenic cytokines by macrophages (Vukanovic J. et al. Cancer Res 55: 1499-1504, 1995).

This large spectrum of serious chronic conditions in which TGF-β overexpression appears to be responsible for tissue damage by scarring, limiting tissue regeneration by inhibition of parenchym proliferation or by protection of pathological cells via immunosuppression illustrates that there is need for a medicament which alleviates such conditions.

It has been suggested by Border et al. (WO 93/10808) and Böttinger et al. (Proc. Natl. Acad. Sci, USA 93:5877-5882, 1996) to treat or arrest the progress of pathologies which are characterized by an accumulation of extracellular matrix components by providing an agent to suppress the activity of transforming growth factor β (TGF-β) protein, leading to fibrosis and angiogenesis. It is suggested to treat a variety of conditions which are connected to these phenomenons, e.g. using TGF-β antibodies, decorin or LAP. However, if the activity of the TGF-β protein is inhibited or suppressed, there is the disadvantage that physiologically circulating TGF-β protein is also affected. Recent reports indicated that total inhibition of TGF-β1 protein may be deleterious since TGF-β1 knock-out mice die shortly after birth due to lack of immunosuppressive activity of TGF-β1 (Shull et al., Nature, 359:693-699, 1992).

Therefore, it is the object of the present invention to provide a medicament for use in reducing or erasing the symptoms arising from excessive accumulation of extra cellular matrix (ECM) which are connected with the said pathological overexpression of TGF-β without adversely affecting the basal expression thereof.

This object has been solved in that use of an agent for the preparation of a medicament is provided according to claim 1. The agent prevents the - pathological - overexpression of TGF-β without affecting the basal expression. This substance is able to prevent AP-1 mediated TGF-β gene expression without substantially affecting the expression regulated by SP 1 sites.

Inhibition of TGF-β is obtained by contacting the affected tissue with such a medicament. The drug may be administered by all conventional methods preferentially dissolved in isotonic saline.

Consequently, this medicament is useful in the treatment of diseases, disorders and conditions which are associated with overexpression of TGF-β, namely pathological accumulation of extracellular matrix (ECM) in cells or tissues.

It should be understood that the present invention is directed to all forms of TGF-β overexpression, although that of TGF-β1 seems to be most important. Consequently, even if in the present description effects are partly described merely in connection with TGF-β1, it should be clear that they are not restricted to this isoform.

The agents or substances of the present invention may be selected under natural, semisynthetic or synthetic, negatively charged oligomers or polymers as defined in claim 1. ("Synthetic" throughout this description means chemical or biochemical/biotechnical production methods). Preferably, they are selected from the glycosaminoglycans of different chemical structures and molecular weights of either natural, semisynthetic or synthetic origin or are synthetic molecules. If the agents or substances are selected under heparins, the heparin is a low anticoagulant heparin having a molecular weight of about 5000 to 6000. Alternatively, the agent can be a dermatan sulfate or a modified dermatan sulfate. The dermatan sulfate may be of high or low molecular weight (e.g. having a m.w. of about 5000-6000 or about 35,000). It may be a low anticoagulant dermatan sulfate. Alternatively, the agent may be a chondroitin sulfate, for example a high molecular chondroitin sulfate (e.g. of about m.w. 24,000) or a modified chondroitin sulfate, a heparan sulfate or a modified heparan sulfate, a hyaluronic acid or a modified hyaluronic acid.

Therefore, although the structure and chemical composition of the glycosaminoglycans seems to make them specifically advantageous for use in the present invention, there is a variety of other agents which may function equally. These agents are preferably related to or derived from glycosaminoglycans. In this connection, it should be mentioned that the structure-function relationship of the glycosaminoglycan activity on TGF-β overexpression is not yet completely known.

In order to assess the effect of glycosaminoglycans, the invention provides a method for evaluating agents for their activity to inhibit TGF-β overexpression.

This is accomplished by an in-vitro assay testing the ability of a substance to inhibit TGF-β overexpression (i.e. AP-1 mediated TFG-β gene expression). In this assay, cultured cells which produce TGF-β in the affected organ are used. The cells are stimulated to produce TGF-β by the addition of an inducing agent; cells and inducing agents differ according to the pathology to be treated. The inhibitory activity of the agent is assessed by the ability to prevent cellular TGF-β overexpression.

In one embodiment, the method comprises the following steps:
(a) treating a sample of target cells, e.g. fat storing cells (FSC) or, preferably, mesangial cells (e.g. porcine mesangial cells) with a substance the presence of which stimulates AP- 1 mediated TGF-β gene expression, e.g. phorbol-myristate-acetate (PMA) in the case of e.g. mesangial cells or FSC or, alternatively, e.g. glucose (in the case of e.g. mesangial cells) in the presence of the glycosaminoglycan to be tested,
(b) transcribing RNA present in the said cells into cDNA
(c) amplifying TGF-β cDNA by PCR
(d) separating the fragments obtained by PCR
(e) quantifying the TGF-β cDNA, and
(f) comparing the reduction of TGF-β cDNA expressed in the target cell sample to that in another target cell sample treated as above but in the absence of the glycosaminoglycan to be tested in step (a).

Use of the phorbolester phorbol-myristate-acetate (PMA) and cultured porcine mesangial cells is preferred. In a specific embodiment, stimulation is performed by 0.5µM of PMA for more than 6 hours. One unit indicates the concentration of the agent needed to reduce the PMA-stimulated cellular TGF-β1 mRNA level by half.

Alternatively, since the glycosaminoglycans of the present invention inhibit TGF-β1 gene overexpression, the potency of the individual agent in question can be assessed by a method for determining its ability to inhibit the previously stimulated TGF-β1 promotor activity.

In one embodiment of this second method, the glycosaminoglycan is tested in an in vitro assay comprising the following steps:
(i) providing a construct containing the TGF-β1 promoter or a biological active fragment thereof, e.g. fragment -453/+11, and a marker gene, e.g. the luciferase gene inserted into a useful vector, e.g. commercially available pGL3basic,
(ii) transfecting a sample of target cells, preferably porcine mesangial cells, with the said vector,
(iii) treating the cells sample with a substance the presence of which stimulates AP-1 mediated TGF-β gene expression, as detailed in the method described above,
(iv) determining the TGF-β promoter activity by measuring the gene marker activity, e.g. luciferase activity, and
comparing the reduction in TGF-β promoter activity in the cell sample to that of a cell sample treated as above but in the absence of the glycosaminoglycan in step (iii).

It is one advantage of the invention that the target cells of the respective tissue may be tested for their ability to be inhibited by a given agent. Such target cells for the in-vitro test system include (examples):

| | |
|---|---|
| target cell: | organ fibrosis: |
| mesangial cell | glomerulosclerosis (of diabetic and other origins) |
| fat storing cell | liver fibrosis/cirrhosis |
| lung fibroblast | lung fibrosis |
| macrophages | glomerulonephritis, cryptogenetic lung fibrosis, peritoneal fibrosis, etc |
| mesothelial cells | peritoneal fibrosis, etc |

In order to stimulate the target cells, phorbol-myristylester may be used which is a well known stimulating agent in the mesangial cell system. In addition to PMA stimulation, the cells may also be stimulated - if known - by the pathologic disease-inducing and/or mediating stimulus e.g. radiation, high glucose concentrations, ethanol, chemicals, etc.
Likewise, the in-vitro cell culture system may also be used to assess the ability of an agent in question to inhibit TGF-β1 overexpression which in turn inhibits parenchym cell proliferation.

Pathological states which can be treated or eventually be prevented by using the medicament of the present invention are characterized by an overexpression of TGF-β and include (1) excessive accumulation of ECM (extracellular matrix). Such conditions include fibrotic diseases, for example, diabetic angiopathies, particularly diabetic nephropathy (glomerulosclerosis), tumor fibrosis, radiation-induced fibrosis, fibrosis of the kidney, liver, lung, bowel, heart, pancreas, peritoneum or other organs, pathological scarring processes, e.g. wound healing, or iatrogenic conditions following irradiation.
It further relates to chronic rejection of kidney graft, and LES nephritis.

It is the advantage of this invention that de novo synthesis of TGF-β of the affected cells but not basal TGF-β expression is inhibited. Therefore unstimulated cells or tissues, i.e. normal, not-injured tissues, are not affected. Furthermore, since the suggested agent(s) do not act at TGF-β protein level, physiologically circulating TGF-β protein, latent or active, and local physiological activation of latent TGF-β are not affected. The specific effect of the suggested treatment is accomplished by specific inhibitory effect of the agent(s) on the AP1-mediated TGF-β1 gene activation while Sp1-mediated gene transcription is not affected.

### Brief description of the drawings

Explanations of abbreviations for glycosaminoglycans and other components mentioned in the figure legends are as described in the examples.

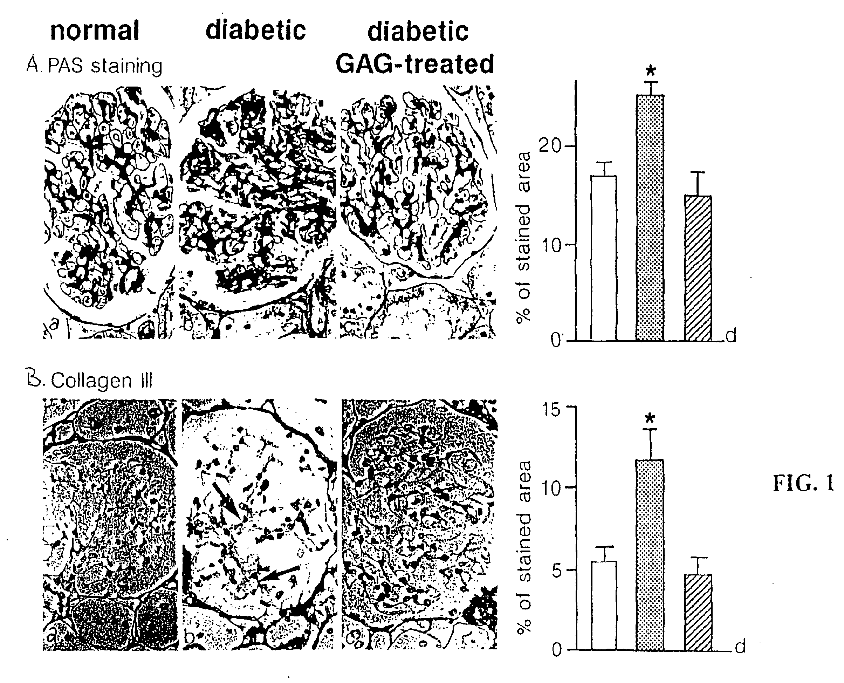

**Figure 1** shows the effect of GAG/mH treatment on renal structure in diabetic rats. (A) Representative photomicrographs of periodic acid Schiff (PAS) stained renal section from normal controls (a), diabetic rats (b) and GAG/mH treated diabetic animals (c). (B) Photomicrographs of renal sections from the same animals immunohistochemically stained for collagen III (a-c). Arrows indicate a marked accumulation of collagen III in the mesangium of diabetic animals. (d) for A and B: Quantification of staining (% of stained area +/- SEM) in glomeruli from normal (open bars), diabetic (solid bars) and GAG/mH-treated (hatched bars) diabetic animals is shown in (d); *p<0.005.

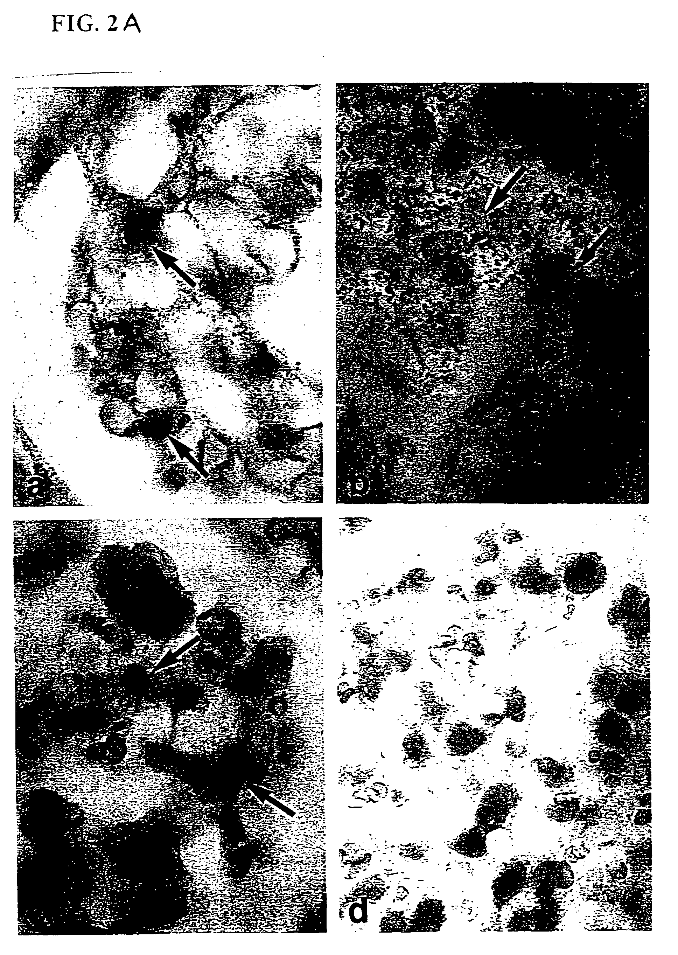

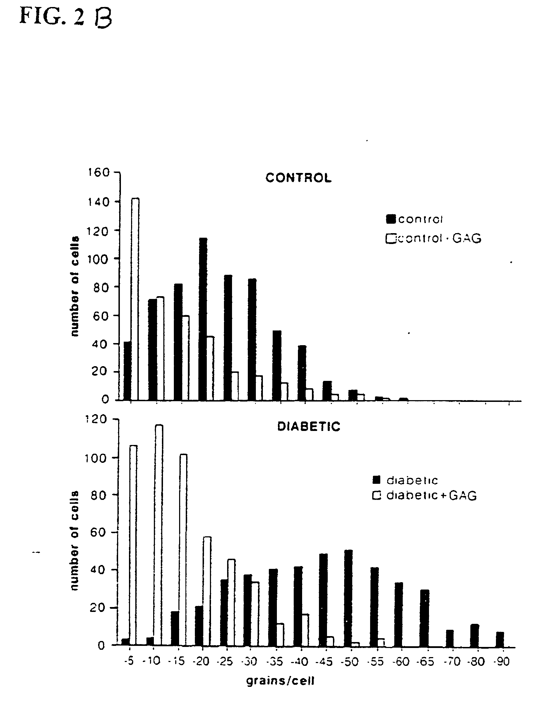

**Figure 2A** shows the effect of GAG/mH therapy on renal TGF-β1 overexpression in long-term diabetic rats. (A) *In-situ* localization of glomerular TGF-β1 mRNA expression in renal sections from control (a), diabetic (b), and GAG/mH-treated diabetic rats (c). For control of the hybridization procedure renal tissue sections were hybridized with a sense riboprobe (d). Only a few positively labelled glomerular cells (arrows) can be localized in normal or GAG/mH-treated diabetic animals. The number of positive cells as well as grain density/cell was increased in untreated diabetic animals (A, b).
**Figure 2B** shows quantitative analysis of the TGF-β1 mRNA signal in glomerular mesangial cells. For evaluation of the *in situ* hybridization experiments the signal was quantified by counting the number of silver grains (see Examples). The distribution of the cells with increasing TGF-β1 mRNA expression is shown for control (upper) and diabetic animals (lower graph). Significant levels of TGF-β1 mRNA are found in normal rats with significant increase in diabetes. A significant reduction in TGF-β1 mRNA signal is found in the GAG/mHtreated diabetic group. In normal rats hybridized with sense riboprobe most cells showed less than 5 grains per cell (not shown).

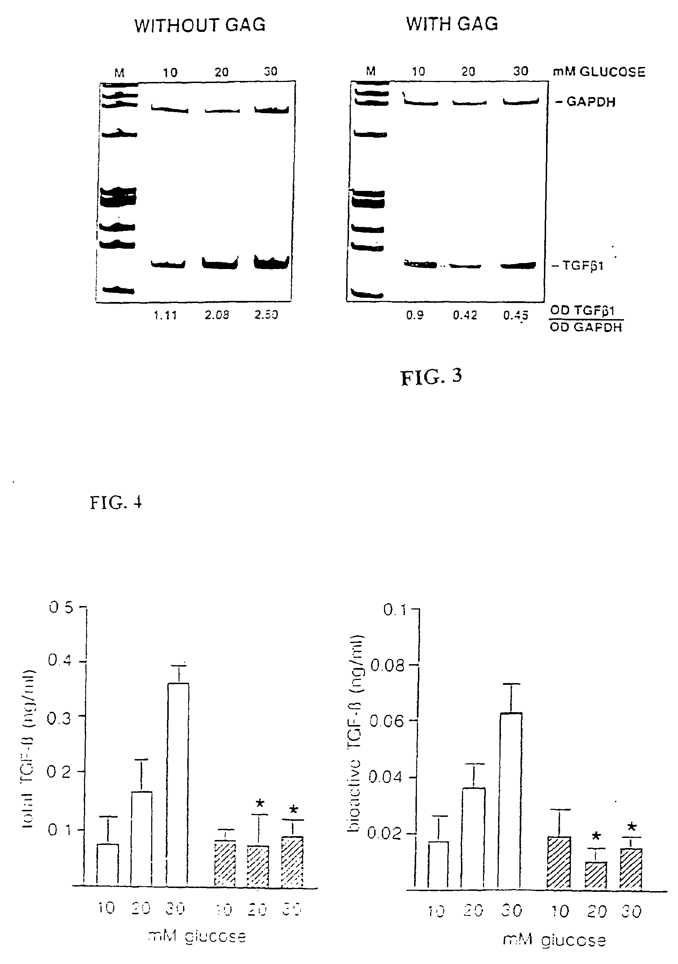

**Figure 3** shows the effect of GAG/mHtreatment on high glucose-induced TGF-β1 mRNA expression in cultured mesangial cells for 48 hrs. Photograph shows PCR products after 30 (TGF-β1) and 24 (GAPDH) cycles, respectively. The numbers below each lane indicate the ratio of TGF-β1/GAPDH optical density (OD) obtained after densitometric scanning of the PCR products. Experiments were performed in triplicate. In the presence of GAG/mH there was no significant difference in TGF-β1 mRNA levels for increasing glucose concentrations.
**Figure 4** shows the effect of GAG/mHtreatment on high glucose-induced TGF-β1 protein production (left) and bioactivity (right). Mesangial cells were incubated with increasing glucose concentrations (10-30 mM) for 40 h without (open bars) or with 10 µg/ml GAG/mH(hatched bars). * p<0.005 compared to values without heparin addition, n=5 dishes.

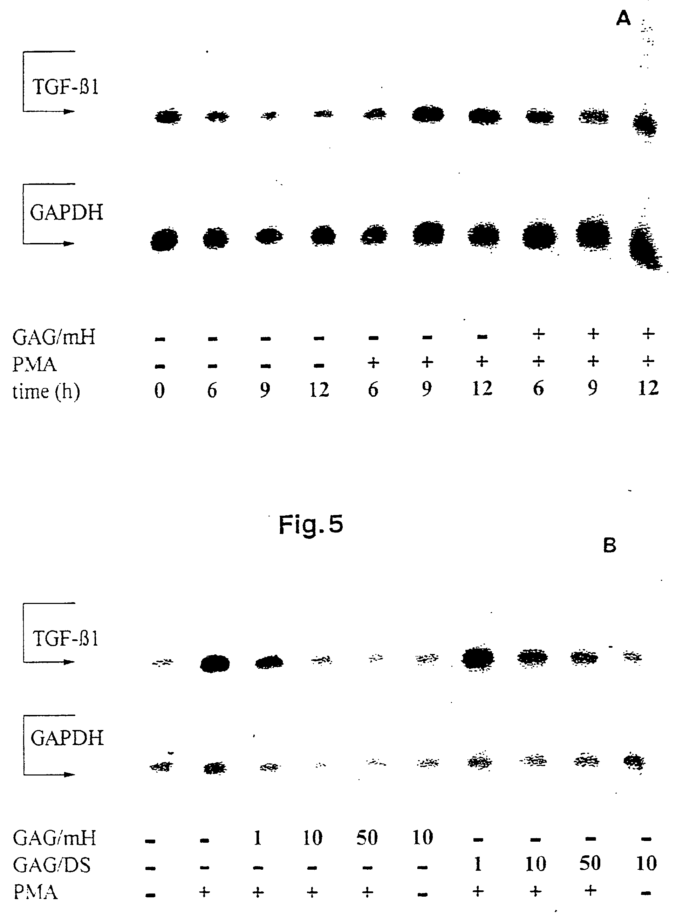

**Figure 5** shows the autoradiograph of an RNase protection assay demonstrating the dose-dependent effect of GAG/mHand dermatan sulfate on PMA-induced TGF-β1 mRNA overexpression. (A) Mesangial cells were grown in standard medium, stimulated with 0.1 µM PMA in the presence or absence of 10 µg/ml GAG/mH, harvested at the time points indicated and RNase protection assay was performed as previously described (Kolm et al. *Am. J. Physiol*. 270: F812-F821, 1996). Labeled cRNA probes for both TGF-β1 and GAPDH were added to each sample. Arrows on the left indicate the shift of full-length cRNA probes for TGF-β1 (356b) and GAPDH (241b) to protected cRNA probes for TGF-β1 (307b) and GAPDH (211b) after RNase A/T1 digestion. No undigested probe is visible in the lanes of the sample. (B) PMA-stimulated mesangial cells were treated with increasing concentrations of GAG/mH (left) or GAG/DS(right) for 12 hours. Mesangial TGF-β1 mRNA levels were determined by RNase protection assay.

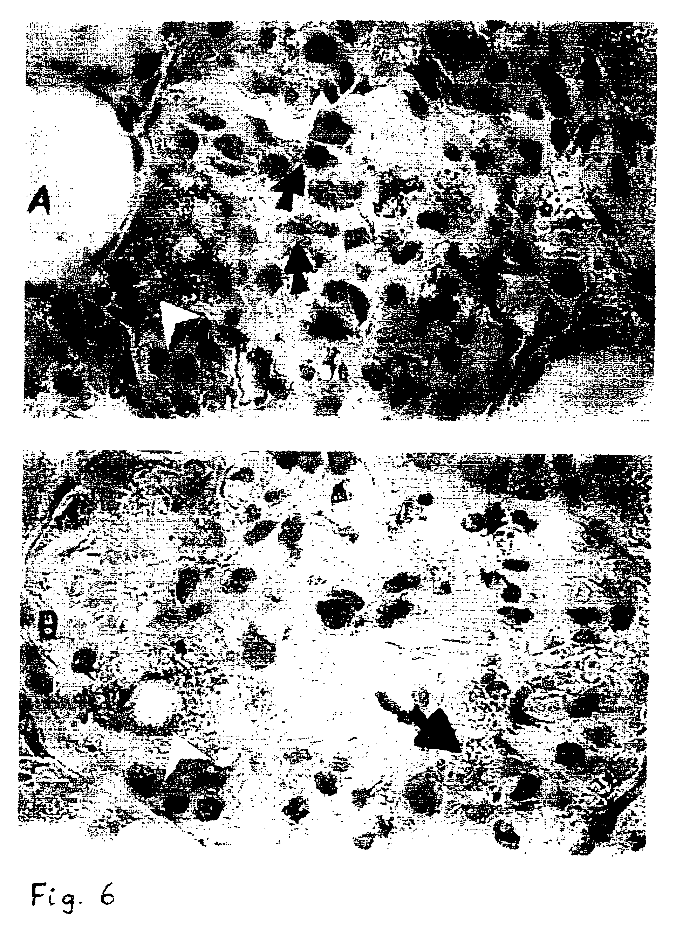

**Figure 6** shows the immunohistochemical micrographs of glomeruli stained with anti-TGF-β1 antibody in the puromycin induced chronic glomerulosclerosis model. (A) Staining of a glomerulus of an untreated nephritic rat. 3 macrophages are evident. (B) Staining of a renal section of a nephritic rat treated with 45 mg GAG/mH/kg body weight. 1 macrophage is evident. The cells were identified as monocyte/macrophages according to their morphology and by comparison with adjacent sections stained with specific macrophage antibodies (ED1 Ab). TGF-β staining is indicated by white arrow heads, and macrophage by black arrow heads.

The contact of the agent with cells or tissues can be performed by addition of a solution of the agent. In vivo, the agent can be administered by well-known procedures like intravenous, intramuscular, sub- or intracutaneous, aerosol, clisma, or oral application. The possible oral application of glycosaminoglycans have been recently described by Jaques et al. *J. Lab. Clin. Med*. 117:122-130, 1991 and by Hiebert et al. *J. Cardiovasc. Pharmacol*. 28:26-29, 1996. The agent may be applied systemically or locally. The amount, the time point and length of application depends on the pathology and individual to be treated.

The effect of various agents, especially of glycosaminoglycans and strongly related compounds, on TGF-β overexpression were studied in-vivo and in-vitro.

Development of diabetic nephropathy is one of the most frequent and serious complications of all types of diabetes and eventually occurs in about 40% of the patients in spite of improving glycemic control. The predominant morphological feature of diabetic nephropathy is the increase in extracellular matrix e.g. thickening of the glomerular basement membrane and particularly indicative, the expansion of the mesangial matrix. Immunohistochemical studies showed that the increase in glomerular PAS-staining is mainly caused by an increase in glomerular, particular mesangial, enhanced deposition of collagens (Nerlich and Schleicher, *Am. J. Pathol*. 139: 889-899, 1991). The morphological changes are paralleled by an increase in urinary protein and albumin excretion rates, widely used indicators of glomerular damage in humans and experimental animals. The similarites of increased glomerular matrix accumulation of glomerulonephritis and diabetic nephropathy suggested the potential involvement of TGF-β in these kidney diseases. Studies with animal models, particularly the streptozotocin-induced diabetes in rat causing diabetic nephropathy (Yamamoto et al. *Proc. Natl. Acad. Sci. USA.* 90:1814-1818, 1993), the puromycin-induced glomerulonephritis in rat (Eddy AA. Exp Nephrol 3: 76-79, 1995; Ding G, van Goor H, Frye J, Diamond JR. Am J Physiol 267: F937-F943, 1994), and adriamycin-induced glomerulosclerosis and interstitial fibrosis (Tamaki et al. *Kidney Int*. 45: 525-536, 1994), showed that TGF-β1 is induced in glomeruli of these animals. (For review of the involvement of TGF-β in diabetic nephropathy see Border et al. *Diabetes*/*Metabolism Reviews* 12: 309.339, 1996). Furthermore, since these animal models share most of the structural and functional (assessed by histology and by determination of albuminuria) features of the human disease; these models are widely used for the investigation of the pathomechanism of the disease and for therapeutic intervention.

Therefore, the effect of agents according to the present invention on renal structural and functional parameters was investigated with these well established animal models.

In addition, the effect of the said agents was also studied with cultured mesangial cells. Numerous studies revealed that mesangial cells respond to hyperglycemia with increased production of extracellular matrix components, e.g. collagens, laminin and fibronectin, and that this increase is caused by high glucose-induced TGF-β1 de novo synthesis (Sharma and Ziyadeh, *Diabetes* 44: 1139-1146, 1996). Therefore, the in-vitro system represents an ideal model for the assessment of medicaments designed to inhibit TGF-β-induced and/or -mediated kidney diseases.

Recent studies on the cell and molecular biology of hepatic fibrogenesis have focused on the fat storing cells (FSC) as the main fibrogenic (precursor) cell type responsible for the extracellular matrix production. However, the full fibrogenic potential of FSC is obtained only if they are activated. This activation includes transition to myofibroblast phenotype, stimulation of the proliferation, induction of gene expression of cytokines and growth factors, particularly TGF-β, and matrix proteins. Therefore, the activated FSC serve as in-vitro model for the investigation of early event in the genesis of liver fibrosis. Activation is achieved by addition of e.g. phorbol-myristate-acetate (PMA) to the culture medium.

Although monocyte/macrophage organ infiltration constitutes a mechanism (both immunological and inflammatory) for healing from a number of injuries, lack of control of it may lead to chronic diseases characterized by the paracrine effect of a number of macrophage cytokines, lesions from reactive oxygen species, eicosanoid-dependent lesions, degradative processes induced by released extracellular proteases (Nikolic-Paterson DJ, Lan HY, Hill PA, Atkins RC. Macrophages in renal injury. Kidney Int 45, Suppl 45; S79-S82, 1994). Among the paracrine effects, noteworthy is the prosclerotic activity of TGF-β released by macrophages on mesangial cells, and the mitogenic effect induced by PDGF, IL-1, IL-6 released by macrophages on mesangial, glomerular epithelial cells, and interstitial renal fibroblasts. Interestingly, the overexpression of TGF-β by the activated macrophages typical of nephritis and other renal diseases is chemotactic for macrophages; thus, a vicious circle occurs which leads to self-maintaining and expanding lesions. Similar scenarios have been described in the lung, for instance in the chronic, idiopathic lung fibrosis, in jatrogenic (toxics, radiations) and occupational lung fibrosis, etc. This pathogenetic cascade has been extensively investigated in the chronic puromycin glomerulosclerosis, a model that is peculiar for two reasons: 1) TGF-β overexpression is due to infiltrating macrophages; 2) mesangial cells are the target of the TGF-β produced by macrophages. In different experimental models the increased TGF-β synthesis is also dependent on mesangial overexpression. Thus, the puromycin model is interesting because it allows to obtain insight essentially on the pathogenetic role of macrophages, and indeed, by blocking macrophage infiltration (i.e., by X ray white cell depletion) in the kidney it is possible to prevent the following renal diseases: glomerular fibrosis, mesangial proliferation, proteinuria, renal failure. Experimental findings on a number of experimental models which formerly were considered non-immunologic (i.e., subtotal renal ablation, ureteral obstruction, puromycin model, etc) were significant in establishing the relationship between the immune system and renal lesions not previously considered to be immunologic in nature). Since macrophage infiltration depends also on the local tissue levels of TGF-β (this growth factor is a potent chemoattractant), the puromycin model is useful to evaluate the role of macrophage released TGF-β on the further recruitment of macrophages; moreover, this model is adequate to evaluate the effect of inhibition on the macrophage paracrine TGF-β loop on mesangial cells (pro-fibrotic phenotype), and indirectly on the many macrophage paracrine loops causing mesangial proliferation. Consequently, also this so called "puromycin model" has been used to establish the usefulness of the agents of the present invention.

### Examples

If not otherwise indicated, the following glycosaminoglycans have the following characteristics:

| | | | |
|---|---|---|---|
| glycosaminoglycan | m.w. (about) | SO₃⁻/CO₂⁻ | % 6-OH |
| GAG/DS, dermatan sulfate | 5000-6000 | 1.7 | 28.3 |
| GAG/mH, heparin | 5000-6000 | 1.3 | 11 |
| GAG/397, dermatan sulfate | 30,000-40,000 | 2 | <3 |
| GAG/296, chondroitin sulfate | 23,000-27,000 | 1.2 | 100 |

### The following abbreviations are used in the examples:

AP-1 activator protein 1
ECM, extracellular matrix
FSC, fat storing cells
GAG(s), glycosaminoglycan(s)
GAPDH, glyceraldehyde-3-phosphate dehydrogenase
mH, modified heparin
PCR, polymerase chain reaction
Sp1, stimulatory protein 1
TGF-β, transforming growth factor B
aPTT actual partial thrombin time
PAS staining perjodate acid Schiff'base staining
PMA phorbol myristate acetate

### Example I

GAG treatment prevents functional and structural changes and TGF-β overexpression in the kidneys of diabetic rats

### 1. Induction of diabetes in rats and treatment with GAG/mH or dermatan sulfate

Diabetes was induced in 6 week old male Sprague-Dawley rats by streptozotocin as described by Gambaro et al. Kidney Int. 46: 797-806, 1994. Half of the diabetic rats (D) and half of control rats (C) were treated subcutaneously with 15 mg/kg body weight/day GAG/mH (mH) or dermatan sulfate (DS) and the remaining half of each group was treated with 2 ml/kg/day of The GAG preparation used in the *in vivo* and *in vitro* studies was a low molecular weight heparin (GAG/mH) (10.4 kDa) chemically modified to yield a preparation with a sulphate/carboxylic ratio of 1.35 showing very low anticoagulant activity (110 µg/ml was necessary to induce a doubling of the aPTT) (Gambaro et al. Kidney Int. 46: 797-806, 1994). In the *in vitro* study a dermatan-sulfate glycosaminoglycan (GAG/DS) preparation characterized as described in (Gambaro et al. Kidney Int. 42: 285-291, 1992) was also used. GAG/DS is a non-anticoagulant glycosaminoglycan with a carbohydrate backbone different from heparin, which, in a previous study was shown to be renoprotective in experimental diabetes (Gambaro et al. Kidney Int. 42: 285-291, 1992). Both drugs were from Alfa Wassermann SpA, Bologna, Italy.

### 2. Immunohistochemical determination of collagen III and TGF-β1 in renal tissue

Kidneys were formaldehyde-fixed and paraffin-embedded. For immunohistochemical studies tissue sections were deparaffinized and treated with PBS containing 0.5% H₂O₂. Mesangial cells were identified with monoclonal anti-rat Thy 1.1 antibody staining obtained from Camon (Wiesbaden, Germany). Immunolocalization of collagen III (Southern Biotechnology Associates, Inc. Oxmoor Boulevard, Birmingham, UK) was performed as described by Nerlich et al. Kidney Int. 45: 1648-1656, 1994. The avidin-biotin complexes were visualized by 3',3'-diaminobenzidine and the sections were counterstained with hematoxylin and evaluated by light microscopy. The sections from all experimental groups were incubated and developed simultaneously. The IgG fraction of rabbit serum was used as negative control. PAS- and immunohistochemical staining was evaluated by morphometric analysis as described Ceol et al. Lab. Invest. 74: 484-495, 1996

### 3. Preparation of TGF-β1 and GAPDH riboprobes

cDNA fragments for TGF-β1 and glyceraldehyde-3-phosphate dehydrogenase (GAPDH) were obtained from mouse spleen and porcine mesangial cell RNA by reverse transcriptase-polymerase chain amplification according to the previously described protocol (Kolm et al. Am. J. Physiol. 270: F812-F821, 1996). The cDNA fragments were then cloned into the transcription vector pGEM3Z (Promega. Madison, USA). The TGF-β1 probe (528bp) is located near the 5'-terminus of the coding sequence (corresponding to aa 32-207). After linearization of the plasmid, single-stranded RNA-probes complementary (anti-sense) or anti-complementary (sense probe, negative control) to cellular mRNA were obtained by run-off transcription using T7 or SP6 polymerase. For *in-situ* hybridization the probes were labeled by digoxigenin using DIG RNA Labelling Kit (Boehringer, Mannheim, Germany)

### 4. In situ hybridization of TGF-β1 on kidney sections and quantitative analysis

Non-radioactive *in situ* hybridization and detection of the bound riboprobes by gold-silver enhancement was performed with digoxigenin-labeled sense and antisense TGF-β1 riboprobes as described (Ceol et al. Lab. Invest. 74: 484-495, 1996). Controls were performed: (a) with sense TGF-β1; (b) with hybridization buffer without probe. *All in situ* hybridization experiments including the silver enhancement were carried out with chamber-slides each containing all experimental conditions in parallel to ensure identical reaction conditions. For quantitative analysis of the *in situ* hybridization data sections from each animal were randomly selected, and grains were counted in glomerular and proximal tubular cells. Only cells with the nucleus and the cytoplasma clearly attributable to a defined cell were evaluated. 10-40 cells per glomerulus and about 8-22 glomeruli per group were counted separately by two investigators resulting in 171-591 cells evaluated per group.

### 5. Results

### Effect of GAG/mH therapy on functional, structural and TGF-β expression changes in the kidneys of diabetic rats.

The glycemia of normal and diabetic animals was not influenced by GAG/mH treatment (Table 1). After 12 months of diabetes duration albuminuria had increased 6-fold. Treatment with GAG/mH prevented the diabetes-induced increase in albuminuria with little effect on controls (Table 1).

**Table 1 Effect of GAG/mH treatment on glycemia and albumin excretion of long-term diabetic rats**

| groups | blood glucose | urinary albumin excretion | n |
|---|---|---|---|
| | mg/dl | mg/24 hr | |
| C | 112.3+/-4.1 | 23.7+/-8.0 | 10 |
| C/mH | 129.8+/-3.9 | 21.5+/-7.0 | 9 |
| D | 590.6+/-19 | 124.3+/-25.9 | 8 |
| D/mH | 580.1+/-31 | 31.8+/-7.1 | 9 |
| C/DS | 119.7+/-5.2 | 25.5+/-8.0 | 10 |
| D/DS | 561.0+/-27 | 24.3/8.1 | 8 |

| | | | |
|---|---|---|---|
| C = normal, D = diabetic rats; mH and DS indicates daily treatment with low anticoagulant, low molecular weight heparin and dermatan sulfate respectively. Values represent mean +/-SEM of 3 independent experiments | | | |

Renal histological analysis in treated and untreated diabetic rats support the beneficial effect of GAG/mH therapy (Fig. 1). Renal sections were treated with periodic acid-Schiff's (PAS) stain and diabetic rats showed an accumulation of PAS stained mesangial matrix (Fig. 1A,b) which was not observed in the controls (Fig. 1A,a) or in the GAG/mH treated animals (Fig. 1 A,c). Similarly, the tubular basement membrane stained more intensely in diabetic animals while these structures appear unchanged in GAG/mH-treated diabetic animals compared to controls. Morphometric quantification of the extent of glomerular PAS-positive matrix confirmed the above observations (Fig. 1A,d). Immunohistochemical analysis of collagen III (Fig. 1B) revealed positive staining in the glomerular mesangial matrix of untreated diabetic rats (Fig. 1B,b) which was significantly lower in normal glomeruli (Fig. 1B,a) and in GAG/mH-treated diabetic rats (Fig. 1B,c). In glomeruli from diabetic rats the collagen III staining was more than two-fold elevated above both normal controls (P < 0.005) and GAG/mH-treated diabetic rats (P<0.001) (Fig. 1B,d). A corresponding increase in diabetes and its amelioration by GAG/mH treatment is also obvious in the peritubular matrix (Fig. 1B). While a clearly increased TGF-β1 staining (more than two-fold) was observed in proximal tubular cells of diabetic animals which was normal in GAG/mH-treated animals (not shown), no significant TGF-β1 staining could be found in the glomerular matrix or in glomerular cells. PAS and collagen III stainings in GAG/mH-treated normal rats were unchanged compared to controls (not shown).

### GAG/mH therapy prevents renal overexpression of TGF-β1 mRNA in long-term diabetic rats.

*In situ* hybridization of renal sections is shown in Fig. 2 for glomerular (A) and tubular (B) TGF-β1 expression. Control sections (Fig. 2A,a) revealed a low intracellular signal for TGF-β1 mRNA which was significantly above the sense control (Fig. 2A,d). In diabetic animals a marked increase in TGF-β1 was seen in all glomerular cells (Fig. 2A,b), particularly in mesangial cells (identified by Thy 1.1 staining). In GAG/mH-treated normal and diabetic rats TGF-β1 expression appeared unchanged when compared to controls (Fig. 2A). Quantitative evaluation of the renal TGF-β1 mRNA expression by counting the grains per glomerular and tubular cell confirmed the TGF-β1 mRNA overexpression. The data for glomerular cells are shown in Fig. 2B. Similarly, TGF-β1 overexpression was observed in tubular epithelial cells of diabetic animals which was prevented by GAG/mH therapy (not shown).

Taken together, our data show that GAG/mH treatment of long-term diabetic rats prevent functional and structural renal changes. Both, the markers for renal damage 1) sclerosis i.e.. increased in PAS- and collagen staining and 2) for renal function i.e. increase in albuminuria are positively influenced by GAG/mH therapy. In addition, the data clearly show the inhibition of TGF-β1 mRNA overexpression by GAG/mH treatment.

### Example II

### GAG treatment prevents hyperglycemia-induced overexpression of TGF-β1 mRNA in mesangial cells

### 1. Mesangial cell culture

Porcine mesangial cells were isolated and characterized as described previously (Kolm et al. Am. J. Physiol. 270: F812-F821, 1996). For experiments cells were grown in standard media (10 to 30 mM glucose) or with PMA (0.1 µM), and without or with varying amounts of GAG for the time period indicated. In case of elevated glucose concentrations mannitol was added to control media. For TGF-β1 bioassay, the conditioned media were centrifuged for 5 min at 4°C. The pellet was discarded and the supernatant was collected, aliquoted, and stored frozen at -20°C until bioassay.

### 2. TGF-β1 bioassay (mink lung cell proliferation assay)

Mink lung epithelial cells (CCL, American type culture collection) were maintained in DMEM (Seromed, Berlin, Germany) with 10% FCS and used for the TGF-β growth inhibition assay essentially as described by Itoh et al. J. Clin. Invest. 91: 2268-2274, 1993. The cells were trypsinized, washed with DMEM, suspended in standard RPMI 1640 with 10% FCS, and seeded at 7 x 10³ cells per 0.2 ml in each well of 96-well dishes. After 3 h mesangial supernatants were added. These supernatants had been extensively dialysed against serum-free RPMI to equilibrate for the different glucose concentrations and were then supplemented with 1 % FCS and 4 mM glutamine before addition to the mink lung cells. For determination of total (latent + active) TGF-β supernatants were heat-activated at 85 °C for 5 min. After 22 h incubation the cells were pulsed with 1.0 µCi ³H-thymidine per well for 2 h, then washed twice with PBS, trypsinized and harvested using a microculture harvesting device and counted. For each assay a standard curve was obtained with 0.01-1 ng/ml of human recombinant TGF-β1 (Gibco, Karlsruhe, Germany) with or without GAG/mH. To neutralize TGF-β activity, a rabbit anti-TGF-β antibody (R&D Systems, Minneapolis, MI, USA) was added at a concentration of 10 mg/ml.

### 3. Determination of mesangial TGF-β1 mRNA by in situ hybridization.

Porcine mesangial cells were cultured to subconfluency on collagen-coated eight-chamber slides, rinsed with PBS containing 1 mM and CaCl₂, 1 mM MgCl_{2,} incubated in 4% paraformaldehyde in RNAse free PBS for 15 min at room temperature and fixed in ethanol/glacial acetic acid (95:5) at -20°C for 5 min. The preparation was rehydrated through alcohol series and the slides were washed with 300 µg/ml heparin in RNAse free PBS. After rinsing in 50 mM Tris-HCl pH 7.5, 5 mM EDTA (5X TE) the slides were treated with 5 µg/ml proteinase K in 1X TE for 15 min at 37 °C. The cells were rinsed with PBS, 0.2% glycine pH 7.5 for 10 min to inhibit the enzymatic activity of proteinase K and in PBS for 5 min at room temperature. After dehydration, hybridization and visualization of the signal was performed with digoxigenin-labeled riboprobes as described previous (Ceol et al. Lab. Invest. 74: 484-495, 1996). For quantification of the data, silver grains in 20 cells per each condition were counted.

### 4. Determination of mesangial TGF-βI mRNA by RT-PCR.

Mesangial cells were grown in 154-cm² dishes, washed, and total RNA was isolated as described (25). 200 ng of total RNA were used to synthesize cDNA with random priming according to the protocol of RNA PCR Kit according to Del Prete et al., Diabetologia 1997, 40: 1449-1454. Four µl of the RT reaction were used to amplify in different tubes TGF-β1 (161 bp) and the housekeeping gene GAPDH (983 bp). The PCR amplification was carried out in a final volume of 50 µl containing 1.5 mM MgCl₂, 0.2 mM dNTP, 2U Taq DNA polymerase from a freshly prepared 28:1 mixture of Taq antibody + Taq polymerase, 0.4 µM primers, 50 mM KCI, 10 mM Tris HCl pH 8. Kinetic analysis was performed starting from 24 to 32 amplification cycles (94°C for 45 s, 60° C for 45 s, 72°C for 2 min) to obtain quantitative data. Primers for TGF-β1 and GAPDH and Taq specific antibody were purchased from Clontech (Palo Alto, USA); Taq polymerase and RNA-PCR Kit were from Perkin Elmer (Branchburg, USA). PCR fragments were separated by polyacrylamide gel electrophoresis and visualized by silver staining. Quantitation was performed by direct densitometric analysis of silver stained bands (IBAS 2000 Kontron). Ratios of Optical Density of TGF-β1 (30 cycles) and GAPDH (24 cycles) PCR products were determined and compared.

### 5. Determination of mesangial TGF-β1 mRNA by RNase protection assay.

Incubations with mesangial cells and RNase protection assays were performed as described previously (Kolm et al. Am. J. Physiol. 270: F812-F821, 1996). For experiments cells were grown in standard media (10 to 30 mM glucose) or with PMA (0.1 µM), and without or with varying amounts of GAG for the time period indicated. For the RNase protection assay a-³²P-UTP (Hartmann, Braunschweig, Germany) 800Ci/mM and the Maxiscript kit (Ambion, Heidelberg, Germany) were used.

### 6. Results

### GAG/mH treatment inhibits high glucose-induced overexpression of TGF-β mRNA, TGF-β-protein and TGF-β bioactivity in cultured mesangial cells.

To elucidate the molecular mechanism of GAG/mH treatment on diabetes-associated TGF-β1 mRNA induction we studied the GAG/mH effect on mesangial cells, the main target cells of diabetes in the glomerulum. Cells were grown in elevated glucose concentrations with and without GAG/mH. Determination of the TGF-β1 mRNA level after reverse transcription and PCR amplification revealed a more than two-fold increase with rising glucose concentrations which was prevented by addition of 10 µg/ml GAG/mH (Fig. 3). Furthermore, the dose-dependent, glucose-induced overexpression of TGF-β1 mRNA was confirmed *by in situ* hybridization in cultured cells. Quantitative analysis of the grains showed a more than threefold increase (10 vs. 30 mM glucose; p<0.005) which was prevented by addition of 10 µg/ml GAG/mH (micrographs not shown).
The effect of GAG/mH on mesangial TGF-β protein expression was also studied. GAG/mH treatment attenuated high glucose-induced mesangial overproduction of TGF-β protein and formation of bioactive TGF-β without affecting basal levels (Fig. 4). To exclude a direct effect of GAG on TGF-β1 action high amounts of GAG (10 µg/ml) were added to active TGF-β1 (1ng/ml) and incubated with mesangial cells.

### GAG/mH and GAG/DS treatment prevents PMA-induced overexpression of TGF-β1 mRNA.

To confirm the potency of GAGs to prevent TGF-β1mRNA overexpression we used PMA, a known, strong inducing agent of TGF-β1 expression. Addition of PMA induced TGF-β1 mRNA four- to five-fold after 9 to 12 hours (Fig. 5A). Coincubation with GAG/mH prevented the PMA-induced TGF-β1 overexpression without affecting basal expression. To demonstrate that the heparin structure and possible associated activities (e.g. anticoagulation, growth factor binding) is not necessary for the inhibitory effect a structurally non-related GAGs, i.e. GAG/DS was used. Presence of increasing amounts of this GAG/DS prevented PMA-induced TGF-β1 mRNA increase dose-dependently, although less efficiently when compared to GAG/mH (Fig. 5B). Again, basal TGF-β1 mRNA levels were unchanged after addition of GAG/DS.
Inhibition experiments were performed by coincubation of bioactive TGF-β1 and mod. heparin and its effect on proliferation of mink lung cells. Presence of GAGs had no effect on the antiproliferative activity of TGF-β1 on these cells. These results showed that 1) mod. heparin and dermatan sulfate do not block TGF-β1 activity indicating that the GAGs do not bind and neutralize TGF-β1 and 2) that the GAGs do not interfere with TGF-β1 receptor binding and theTGF-β1 mediated TGF-β1 signal transduction. These results show that GAGs act at the nucleic acid level and not at the protein level
Taken together, these data show that high glucose- or PMA-induced TGF-β1 mRNA overexpression may be dose-dependently inhibited by GAG, particularly mH and DS.

### Example III

### GAG treatment prevents TGF-β1 expression in at-storing cells (FSC) from human liver

### 1. Preparation of FSC-cells

Human FSC wer isolated according to the method of Friedman and Roll *Anal. Biochem.* 161: 207-218, 1987.FSC were plated in plastic tissue cultures flasks (NUNCKarIsruhe,Germany) at a density of 0.5-1x10⁶ cells/ml and incubated at 37°C in 5%CO2. The medium was replaced after 24 hours and every 72 hours.The typical cell morphology, stellate-like appearance, was studied with an inverted microscope (Axiovert, Zeiss, Oberkochen, Germany). The characteristic vitamin A fading green-blue fluorescence was detected when cells were exposed to UV-light at 330 nm.
FSC were stimulated with 0.5µM PMA for 8 hours in the presence and absence of different GAG preparations. Cells were harvested and RT-PCR was performed as described in Example II. PCR products of TGF-β1 and GAPDH were separated on SDS-gel electrophoresis and siver stained. Band intensity was quantified by scanning of the gels.

### 2. Inhibition of TGFβ1 overexpression by GAG treatment

Table 2 shows that the presence of 10 µg/ml GAG reduces the mRNA level of TGF-β1 stimulated by PMA back to basal levels. The mRNA level of the house-holding enzyme GAPDH is not affected.

### Table 2

Effect of GAG on PMA-induced TGF-β1 mRNA overexpression in fat storing cells

| Additions | % of control |
|---|---|
| none | 100 |
| PMA | 236 |
| PMA+GAG/mH | 86 |
| PMA+ GAG/DS | 112 |

FSC from liver were stimulated with PMA and TGF-β1 and GAPDH levels were measured by RT-PCR and quantified by scanning of the stained gels. Results are expressed as % of control. Values represent the mean of 2 determinations.

### Example IV

### GAG treatment reduces the glomerular infiltration of macrophages and inhibits the synthesis of TGF-β1 and latent TGF-β binding protein.

### 1. Induction of glomerulosclerosis

Glomerulosclerosis was induced in 24 male Wistar rats (200-225g) by i.v. injection of 50 mg/kg body weight puromycin (PAN) (Sigma, Milan Italy) followed at weekly interval by 2 more i.v. administration (20 mg/kg body weight). After the last PAN treatment rats were divided in 3 groups and treated with daily s.c. injections of 0.5 ml of 0.9% NaCl without (PAN/0) or with 30 or 45 mg/kg body weight modified heparin (PAN/30 and PAN/45). The modified heparin was a chemically desulphated (sulfate/carboxyl ratio 1.34/1) low molecular weight heparin (MW 5800 Da) with low anticoagulant activity. Untreated rats served as contols (C). After 90 days rats were sacrificed. 24 hour urine was collected from day 89 and total protein and albumin was determined as described by Gambaro et al. Kidney Int. 46: 797-806, 1994.

### 2. Immunohistochemistry of TGF-β1 and collagen

5 µm section were prepared from formaldehyde-fixed paraffin-embedded renal tissues. Hematoxylin-eosin, periodic acid-Schiff-staining and immunostaining for collagen IV, TGF-β1 and ED-1 was performed as described by Nerlich et al. Kidney Int. 45: 1648-1656. 1994. The ED-1 antibody which specifically recognizes cytoplasmic in monocytes/macrophages (Dijkstra et al. Immunology 54: 589-599, 1985) was from Serotec, Oxford, UK. TGF-β1 antiserum was from Santa Cruz Biotechnology Inc., Santa Cruz, USA and Collagen IV antiserum from Eurodiagnostics, Leiden, Netherlands. A semi-quantitative sclerosis score was determined essentially according to Yoshida et al. Kidney Int. 36: 626-635, 1989. Briefly, 1 +, 2+, 3+, 4+ corresponds to 25%, 50%, 75% and 100% of the glomerular section on sclerosis. respectively. Macrophage infiltration was assessed by counting the number of ED-1 positive cells per glomerulus; the clasification was 1, 2. 3, and 4, for 1, 2, 3, and 4 or more ED-1 positive cells per glomerulus.

### 3. Results

Induction of aminoglycoside nephrosis by PAN resulted in increased proteinuria and particularly increased (10-fold) albuminuria when compared to controls (Table 3). Treatment of the rats daily with 30 and 45 mg mod. heparin/kg body weight reduced these urinary excretion dose-dependently. Accordingly the glomerulosclerosis score of untreated PAN animals of 58 was significantly reduced to 36 and 21 by treatment with increasing heparin doses (Table 3). Furthermore, the markedly increased glomerular infiltration of macrophages/ monocytes was reduced in heparin-treated animals.

### Table 3

Effect of modified heparin treatment on renal parameters of nephritic rats

### ("Modified heparin" means the modified heparin (GAG/mH) according to Example I)

| group | urinary protein | urinary albumin | glomerulosclerosis | infiltrated macrophages |
|---|---|---|---|---|
| | mg/24 hr | mg/24 hr | score | mean/glomerulus |
| C | 46+/-3 | 14+/-3 | 3+/-1.5 | 0.03+/-0.01 |
| PAN/0 | 167+/-15 | 154+/-15 | 58+/-8 | 0.58/+/-0.10 |
| PAN/30 | 117+/-15 | 107+/-16 | 36+/-11 | 0.32+/-0.06 |
| PAN/45 | 108+/-12 | 95+/-18 | 21+/-7 | 0.22+/-0.05 |

Rats were made nephritic by PAN injection and subsequently treated with daily s.c. injections of 0.9% NaCl containing 0, 30 or 45 mg mod. heparin/kg body weight i.e. group PAN/0, PAN/30 and PAN/45. Untreated animals served as controls. 24 hour urinary excretion of protein and albumin was determined, and glomerular sclerosis score was obained by assessing the percentage of the glomerular section area on sclerosis. Glomerular monocyte/macrophage infiltration was assessed by ED-1 staining. Values represent mean +/- SEM of 3 independent experiments

The macrophages/monocytes were intensely stained by the anti-TGF-β1 antisera (Fig. 6) while significantly reduced staining was observed in the modified heparin-treated animals. Deposition of TGF-β1 in the extracellular mesangial matrix was faint in the nephritic animals and virtually absents in the modified heparin-treated group (Fig.6B). These immunostainings were virtually negative in all control animals. Collagen IV immunostaining showed heavy peri-macrophagic deposition, and intense labeling with mesangial pattern in the nephritic animals, while the immunolabeling was modest in peri-macrophagic and mesangial areas in mod-heparin-treated animals (data not shown). No mortality or bleeding was observed in any animal, except occasional small hematomas at the site of heparin injection.

### Example V

To assess the inhibitory activity of the GAG action on TGF-β1 overexpression an in vitro assay was established. In this assay structurally distinct GAGs were investigated for their activity to inhibit stimulated TGF-β1 mRNA overexpression in cultured target cell. For the development of glomerulosclerosis (e.g. diabetes) mesangial cells are one major pathological target. Mesangial cells were isolated, characterized and grown as decribed by Kolm et al. *Am. J. Physiol.* 270: F812-F821, 1996. Cells were stimulated with PMA in the presence and absence of a variety of 10 µg/ml of GAGs and related compounds. Cellular mRNA TGF-β1 levels were determined as decribed in Example II.
The separation and the precise quantification of the PCR products was performed on a Biofocus 3000 capillary electrophoresis system equipped with a laser-induced fluorescence detector (Bio-Rad, Munich, Germany) using an argon-ion laser. Excitation power was approximately 3.5 mW at λ = 488 nm, and fluorescence emission was detected after passing through a 520 nm band pass filter. The separations were performed in a poly(N-acryloyl-aminoethoxyethanol)-coated fused silica capillary of 75 µM inner diameter and 31 cm length. The separation buffer consisted of 90 mM Tris-borate, 2mM EDTA and hydroxyethylcellulose (Sigma, St. Luois, Mo, USA). For on-column DNA fluorescence labeling, 1µl Syber Green I (Molecular probes, Eugene, Or, USA) was addes per ml. The PCR samples were diluted 1:30 with water, loaded electrophoretically at a constant voltage of 12 kV and 20 °C. The migration time for the PCR products was 5.2 min for TGF-β1 (161bp) and 6.5 min for GAPDH (983 bp) All peak areas were calculated automatically using Biofocus Integration Software.

### Results

**Table 4 Effect of GAG on PMA-induced TGF-β1 mRNA overexpression in porcine mesangial cells**

| Additions | % of control |
|---|---|
| none | 100 |
| 30 mM glucose | 167 |
| 30 mM glucose +mH | 81 |
| 30 mM glucose + DS | 108 |
| PMA | 312 |
| PMA+mH | 91 |
| PMA+ DS | 109 |
| PMA+ GAG/392 | 122 |
| PMA+ GAG/296 | 147 |
| PMA + chitosan-6-sulfate | 116 |

Mesangial cells were stimulated with glucsoe or with PMA and TGF-β1 and GAPDH levels were amplified by RT-PCR. Resulting PCR product were quantified by separating and measuring the fluorescence on capillary electrophoresis. Results are expressed as % of control. Values represent the mean of 2 determinations.

### Example VI

Target cells were obtained as described in Example V. A construct of the TGF-β1 promotor and luciferase was obtained by ligation of the human TGF-β1 promotor fragment -453/+11 inserted in pGL3basic (Promega, Germany) upstream the luciferase gene. Cells were transfected with the TGF-β1 promotor-Luciferase construct using the calcium phosphate coprecipitation method. After transfection cells were treated with 6 or 30 mM glucose for 40 hours or with 0.5 µM PMA for 9 hours. Total cellular protein was determined by the protein kit obtained from Bio-Rad (Munich, Germany). After 9 or 40 hours the cells were harvested and TGF-β1 promotor activity was determined by measuring the luciferase activity with the Luminometer from Berthold (Wildbach. Germany).

### Results

**Table 5**

| Stimulus | GAG/mH | % of control |
|---|---|---|
| | 10 µg/ml | |
| 6mM glucose | - | 100 |
| 6mM glucose | + | 87 |
| 30mM glucose | - | 163 |
| 30mM glucose | + | 78 |
| 0.1 µM PMA | - | 298 |
| 0.1 µM PMA | + | 91 |

Effect of GAG on glucose- or PMA-stimulated TGF-β1 promotor activity TGF-β1 promotor activity was determined by using the TGF-β1 promotor-luciferase construct. Values are given as % of control +/- SEM.

The data indicate that upon stimulation of the mesangial cells either by high glucose levels or by PMA TGF-β1 promotor activity is increased indicating enhanced gene transcription activity. Presence of 10 µg/ml GAG completely inhibited TGF-β1 gene overexpression.

## Claims

1. Use of an agent which is able to prevent AP-1 mediated TGF-β gene expression without substantially affecting the expression regulated by SP1 sites, the agent being selected term the group of a low anticoagulant heparin, having a molecular weight of about 5000 to 6000, dermatan sulfate, modified dermatan sulfate, chondroitin sulfate, modified chondroitin sulfate, heparan sulfate, modified heparan sulfate, hyaluronic acid, and modified hyaluronic acid, all substances being natural, modifications from natural substances or substances synthetically prepared, either by chemical or biochemical methods,
for the preparation of a medicament for the treatment of disorders selected from fibroses and organ failures derived therefrom, connected with pathological overexpression of TGF-β gene and excessive accumulation of extracellular matrix (ECM), the medicament being intended to reduce or erase the symptoms arising from excessive accumulation of extracellular matrix (ECM), - except use of sulodexine, low molecular weight heparin derivatives obtained by chemical or enzymatic depolymerization, chemically modified heparin derivatives and low molecular weight dermatan sulfates obtained by chemical or enzymatic depolymerization for the treatment of diabetic nephropathy.

2. Use of an agent according to claim 1, **characterized in that** the agent is a negatively charged oligomer or polymer, preferably a glycosaminoglycan.

3. Method for testing the ability of a glycosaminoglycan to prevent AP-1 mediated TGF-β expression without substantially affecting the expression regulated by SP1 sites, comprising the following steps:
(a) treating a sample of target cells with a substance the presence of which stimulates AP-1 mediated TGF-β gene expression in the presence of the glycosaminoglycan to be tested,
(b) transcribing RNA present in the said cells into cDNA
(c) amplifying TGF-β cDNA by PCR
(d) separating the fragments obtained by PCR
(e) quantifying the TGF-β cDNA, and
(f) comparing the reduction of TGF-β cDNA expressed in the cell sample to that in another target cell sample treated as above but in the absence of the glycosaminoglycan to be tested in step (a).

4. Method according to claim 3, **characterized in that** the target cells are porcine mesangial cells and/or the substance the presence of which stimulates AP-1 mediated TGF-β gene expression is phorbol-myristate-acetate or glucose and/or quantification of the TGF-β cDNA is performed using fluorescence labelling.

5. Method for testing the ability of a glycosaminoglycan to prevent AP-1 mediated TGF-β expression without substantially affecting the expression regulated by SP1 sites, comprising the following steps:
(i) providing a construct containing the TGF-β1 promoter or a biological active fragment thereof, and a marker gene, inserted into a useful vector
(ii) transfecting a sample of target cells with the said vector,
(iii) treating the cell sample with a substance the presence of which stimulates AP-1 mediated TGF-β gene expression in the presence of the glycosaminoglycan to be tested,
(iv) determining the TGF-β promoter activity by measuring the activity of the marker gene,and
(v) comparing the reduction in TGF-β promoter activity in the target cell sample to that of another target cell sample treated as above but in the absence of the glycosaminoglycan to be tested in step (iii).

6. Method according to claim 5, **characterized in that** the target cells are porcine mesangial cells and/or the biological active fragment of TGF-β1 promoter is fragment -453/+11 and/or the marker gene is luciferase gene and/or the substance the presence of which stimulates AP-1 mediated TGF-β gene expression is phorbol-myristate-acetate or glucose.

## Revendications

1. Utilisation d'une substance capable d'empêcher l'expression du gène de TGF-β sous control d' AP-1 sans affecter sensiblement l'expression de TGF-β sous contrôle de sites SP1, la substance sélectionnée (du groupe existant) consistant d'une héparine à basse activité anticoagulante, d'un poids moléculaire d'environ 5000 à 6000, de sulfate de dermatan, de sulfate de dermatan modifié, de sulfate de chondroitine, de sulfate de chondroitine modifié, de sulfate d'héparan, de sulfate d'héparan modifié, d'acide hyaluronate et d' acide hyaluronate modifié, toutes ces substances étant naturelles, modifiées à partir de substances naturelles ou préparées synthétiquement par des méthodes chimiques ou biochimiques,
pour la préparation d'un médicament pour le traitement de dérangements sélectionnés de fibroses et de dysfonctionnement d'organes engendrés par celles-ci, ayant en commun d'être connectés avec une hyperexpression pathologique du gène de TGF-β et une accumulation excessive de la matrice extracellulaire (MEC), le médicament étant conçu pour diminuer ou éradiquer les symptômes créés par l'accumulation excessive de la matrice extracellulaire (ECM) - excepté l'utilisation de Sulodexine, de dérivés de l'héparine de bas poids moléculaire obtenus par dépolymerisation chimique ou enzymatique, de dérivés de l'héparine modifiés chimiquement et les sulfates de dermatan de bas poids moléculaire obtenus par dépolymerisation chimique ou enzymatique pour le traitement de la néphropathie diabétique.

2. Utilisation d'une substance selon la revendication 1, **caractérisée par le fait que** la substance soit un oligomère ou polymère de charge négative, préférablement un glycosaminoglycane.

3. Procédé pour tester la capacité d'un glycosaminoglycane à empêcher l'expression du gène de TGF-β sous le contrôle d'AP-1 sans affecter sensiblement l'expression régulée par les sites SP1, comprenant les étapes suivantes :
(a) traîtement d'un échantillon de cellules cibles avec une substance qui stimule l'expression du gène de TGF-β en présence du glycosaminoglycane à tester,
(b) transcription de l'ARN présent dans la cellule susnommée en ADNc
(c) amplification de l'ADNc de TGF-β par PCR
(d) séparation des fragments obtenus par PCR
(e) quantification de l'ADNc de TGF-β et
(f) comparaison de la réduction de l'ADNc de TGF-b exprimé dans les cellules échantillon à celle observée dans un autre échantillon de cellules cibles traité comme ci-dessus mais en l'absence du glycoaminoglycane à tester dans l'étape (a).

4. Procédé selon la revendication 3, **caractérisé par le fait que** les cellules cibles sont des cellules mésangiales porcines et/ou que la substance dont la présence stimule l'expression du gène de TGF-b sous le contrôle d'AP-1 est l'acétate myristate de phorbol ou le glucose et/ou la quantification de l'ADNc de TGF-b est faite par marquage fluorescent.

5. Procédé pour tester la capacité d'un glycosaminoglycane à empêcher l'expression du gène de TGF- β sous le contrôle d'AP-1 sans affecter sensiblement l'expression régulée par les sites SP1, comprenant les étapes suivantes :
(i) préparation d'un construct comprenant le promoteur de TGF-β1 ou un fragment biologiquement actif de celui-ci et d'un gène indicateur inséré dans un vecteur utile,
(ii) transfection d'un échantillon de cellules cibles avec le dit vecteur,
(iii) traitement d'un échantillon de cellules avec une substance qui stimule l'expression du gène de TGF-b sous löe contrôle d'AP-1 en présence du glycoaminoglycane à être testé
(iv) détermination de l'activité du promoteur de TGF-β en mesurant l'activité du gène indicateur et
(v) comparaison de la réduction de l'activité du promoteur de TGF-β dans l'échantillon de cellules cibles à celle observée dans un autre échantillon de cellules cibles traité comme ci-dessus mais en l'absence du glycoaminoglycane à être testé à l'étape (iii).

6. Procédé selon la revendication 5, **caractérisé par le fait que** les cellules cibles sont des cellules mésangiales porcines et/ou le fragment biologiquement actif du promoteur de TGF-β1 est le fragment -453/+11 et/ou le gène marqueur est le gène de la luciférase et/ou que la substance dont la présence stimule l'expression du gène de TGF- β sous le contrôle d'AP-1 est l'acétate myristate de phorbol ou le glucose.

## Patentansprüche

1. Verwendung eines Agenz, das fähig ist, die AP-1 vermittelte TGF-β Expression zu verhindern, ohne dabei die durch SP 1 regulierte Expression wesentlich zu beeinflussen, wobei das Agenz ausgewählt ist aus der folgenden Gruppe: Heparin mit niedriger antikoagulanter Wirkung mit einem Molekulargewicht von ungefähr 5000 bis 6000, Dermatansulfat, modifiziertes Dermatansulfat, Chondroitinsulfat, modifiziertes Chondroitinsulfat, Heparansulfat, modifiziertes Heparansulfat, Hyaluronsäure und modifizierte Hyaluronsäure, wobei alle Substanzen natürlich, Modifikationen von natürlichen Substanzen oder durch chemische oder biochemische Methoden synthetisch hergestellte Substanzen sind,
für die Herstellung eines Medikaments zur Behandlung von Störungen, ausgewählt unter Fibrosen und daraus resultierenden Organversagen, die mit pathologischer Überexpression des TGF-β Gens und exzessiver Akkumulierung von extrazellulärer Matrix (ECM) verbunden sind, wobei das Medikament dafür vorgesehen ist, die Symptome, die von der exzessiven Akkumulierung extrazellulärer Matrix (ECM) herrühren, zu reduzieren oder völlig zu beseitigen, - ausgenommen die Verwendung von Sulodexine, Derivaten von Heparin mit niedrigem Molekulargewicht, die durch chemische oder enzymatische Depolymerisation erhalten wurden, chemisch modifizierten Heparinderivaten und Dermatansulfaten mit niedrigem Molekulargewicht, die durch chemische oder enzymatische Depolymerisation erhalten wurden, für die Behandlung der diabetischen Nephropathie.

2. Verwendung eines Agenz nach Anspruch 1, **dadurch** charakterisiert, dass das Agenz ein negativ geladenes Oligomer oder Polymer, vorzugsweise ein Glycosaminoglycan, ist.

3. Verfahren zum Testen der Fähigkeit eines Glycosaminoglycans, die AP-1 vermittelte TGF-β-Expression zu verhindern, ohne dabei die durch SP1 regulierte Expression wesentlich (nachteilig) zu beeinflussen, umfassend die folgenden Schritte:
(a) Behandeln einer Probe von Zielzellen in Anwesenheit des zu testenden Glycosaminoglycans mit einer Substanz, deren Anwesenheit die AP-1 vermittelte TGF-β Genexpression stimuliert;
(b) Transkribieren der in der besagten Zelle vorhandenen RNA in cDNA
(c) Amplifizieren der TGF-β cDNA durch PCR
(d) Trennen der durch PCR erhaltenen Fragmente
(e) Quantifizieren der TGF-β cDNA, und
(f) Vergleichen der Reduktion der TGF-β cDNA, die in der Zellprobe exprimiert wurde, mit derjenigen in einer anderen Zielzelle, welche wie oben beschrieben aber ohne Zusatz des zu testenden Glycosaminoglycans in Schritt (a) behandelt wurde.

4. Verfahren gemäß Anspruch 3, **dadurch** charakterisiert, dass die Zielzellen Schweinemesangienzellen sind und/oder dass die Substanz, deren Anwesenheit die AP-1 vermittelte TGF-β Genexpression stimuliert, Phorbol-Myristat-Acetat oder Glucose ist und/oder dass die Quantifizierung der TGF-β cDNA mittels Fluorenszenzmarkierung durchgeführt wird.

5. Verfahren zum Testen der Fähigkeit eines Glycosaminoglycans, die AP-1 vermittelte TGF-β Expression zu verhindern, ohne dabei die durch SP 1 regulierte Expression wesentlich (nachteilig) zu beeinflussen, umfassend die folgenden Schritte:
(i) Bereitstellen eines Konstrukts, das den TGF-β1 Promotor oder ein biologisch aktives Fragment daraus und ein Markergen beinhaltet, das in einen gebrauchsfähigen Vector insertiert ist,
(ii) Transfizieren einer Probe von Zielzellen mit besagtem Vector,
(iii) Behandeln der Zellprobe mit einer Substanz, deren Anwesenheit die AP-1 vermittelte TGF-β Genexpression in Anwesenheit des zu testenden Glycosaminoglycans stimuliert,
(iv) Bestimmen der TGF-β Promotoraktivität durch Messen der Aktivität des Markergens, und
(v) Vergleichen der Reduktion der TGF-β Promotoraktivität in der Zielzellprobe mit der einer anderen Zielzellprobe, die wie oben aber in Abwesenheit des zu testenden Glycosaminoglycans in Schritt (iii) behandelt wurde.

6. Verfahren gemäß Anspruch 5, **dadurch** charakterisiert, dass die Zielzellen Schweinemesangienzellen sind und/oder das biologisch aktive Fragment des TGF-β Promoters das Fragment -453/+11 ist und/oder das Markergen das Luziferasegen ist und/oder dass die Substanz, deren Anwesenheit die AP-1 vermittelte TGF-β Genexpression stimuliert, Phorbol-Myristat-Acetat oder Glucose ist.
